# EUROPEAN PATENT SPECIFICATION

(11) **EP 4 324 510 B1**
(45) Date of publication and mention of the grant of the patent: **05.03.2025**
(21) Application number: 23191421.9
(22) Date of filing: 14.08.2023
(51) Int. Cl.: A61N 1/05, A61N 1/375, A61B 17/00

(54) **BIOSTIMULATOR TRANSPORT SYSTEM HAVING DRIVE BELT**
BIOSTIMULATORTRANSPORTSYSTEM MIT ANTRIEBSRIEMEN
SYSTÈME DE TRANSPORT DE BIOSTIMULATEUR AYANT UNE COURROIE D'ENTRAÎNEMENT

(30) Priority: 17.08.2022 US 202263398820 P; 07.08.2023 US 202318231178
(43) Date of publication of application: 21.02.2024
(73) Proprietor: Pacesetter, Inc., Sylmar, CA 91342 (US)
(72) Inventor: CHANTASIRIVISAL, Steve, Sylmar, CA 91342 (US); VICTORINE, Keith, Sylmar, CA 91342 (US); NIX, Kyle J, Sylmar, CA 91342 (US); MEDINA, Christian, Sylmar, CA 91342 (US)
(74) Representative: Barker Brettell Sweden AB

(56) References cited:
- US-A1- 2016 015 983
- US-A1- 2016 310 723
- US-A1- 2017 209 688
- US-A1- 2017 281 952
- US-A1- 2017 312 496
- US-A1- 2018 264 273
- US-B2- 10 765 872
- US-B2- 11 219 760

## Description

### TECHNICAL FIELD

The present disclosure relates to biostimulator systems. More specifically, the present disclosure relates to biostimulator transport systems for transporting leadless biostimulators useful for septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. US 2017/0281952 discloses extraction devices for extracting chronically implanted devices such as leadless cardiac pacemakers. US 11,219,760 discloses systems for positioning a leadless pacing device in cardiac tissue. US 10,765,872 discloses systems for delivering and retrieving a leadless pacemaker. A delivery system can be used to deliver the leadless cardiac pacemakers to the target anatomy. US 2016/0310723, US 2016/0015983 and US 2017/0209688 disclose delivery tools for implantable medical devices.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch (LBB) is an alternative to His-bundle pacing. Pacing at the LBB involves pacing past the His-bundle toward the right ventricle apex. More particularly, a pacing site for LBB pacing is typically below the His-bundle, on the interventricular septal wall near the tricuspid valve and pulmonary artery outflow track.

### SUMMARY OF THE DESCRIPTION

Existing leadless pacemakers may not fit, or may interfere with heart structures, when placed at a pacing site for left bundle branch (LBB) pacing. More particularly, existing leadless pacemakers have bodies that are long and rigid and, when implanted at the interventricular septal wall, could extend into contact with the cardiac tissue of a ventricular free wall or the tricuspid valve. The long and rigid body of existing leadless pacemakers could also become tangled within chordae tendinae. Furthermore, a proximal end of the existing leadless pacemakers may flail within the heart chamber as the heart beats, causing cyclical contact with adjacent heart structures. Such contact could interfere with heart function.

A leadless biostimulator that can be engaged to the interventricular septal wall, e.g., within a right ventricle, to pace the LBB without interfering with adjacent structures of the heart may include a flexible extended electrode. The flexible extension electrode can extend from a biostimulator housing to allow a pacing electrode to be located at an upper region of a septal wall. A housing of the biostimulator may be located near an apex of the right ventricle. Transportation to or from the right ventricle can be facilitated by a biostimulator transport system, as described below.

The present invention is defined in the independent claim. Further embodiments of the invention are defined in the dependent claims.

A biostimulator transport system is described. The biostimulator transport system includes a sleeve having a sleeve lumen, a support member extending through the sleeve lumen to a distal member end, and a belt extending longitudinally through the sleeve lumen between the sleeve and the support member. The belt can extend to a loop distal to the distal member end. For example, the loop can be a portion of the belt at which belt legs extend proximally back along the support member. The loop can define a gap between a socket of the support member at the distal member end and the belt.

A biostimulator system is also described. The biostimulator system includes a biostimulator, and the biostimulator transport system. The biostimulator includes a pacing electrode electrically connected to pacing circuitry contained within a housing of the biostimulator. A portion of the biostimulator, e.g., an articulation of a flexible extension electrode, can pass through the loop between the belt and the distal member end. The belt can be moved, e.g., by a drive mechanism, to spin the biostimulator within the socket at the distal member end. The pacing electrode of the biostimulator may be screwed into a target tissue when the biostimulator is rotated by the belt with the pacing electrode engaged to the target tissue.

The above summary does not include an exhaustive list of all aspects of the present description. It is contemplated that the description includes all systems that can be practiced from all suitable combinations of the various aspects summarized above, as well as those disclosed in the Detailed Description below and particularly pointed out in the claims. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross-section of a patient heart illustrating an example implantation of a biostimulator in a target anatomy, in accordance with an embodiment.
FIG. 2 is a perspective view of a biostimulator system, in accordance with an embodiment.
FIG. 3 is a cross-sectional view of a biostimulator retaining within an extended sleeve of a biostimulator transport system, in accordance with an embodiment.
FIG. 4 is a cross-sectional view, taken about line A-A of FIG. 3, of a biostimulator transport system, in accordance with an embodiment.
FIG. 5 is a cross-sectional view, taken about line B-B of FIG. 3, of a biostimulator transport system, in accordance with an embodiment.
FIG. 6 is a cross-sectional view of a biostimulator exposed from a retracted sleeve of a biostimulator transport system, in accordance with an embodiment.
FIG. 7 is a top view of a biostimulator exposed from a retracted sleeve of a biostimulator transport system, in accordance with an embodiment.
FIG. 8 is a side view of a biostimulator exposed from a retracted sleeve of a biostimulator transport system, in accordance with an embodiment.
FIG. 9 is a flowchart of a method of implanting a biostimulator for septal pacing.
FIG. 10 is a diagrammatic view of an operation of advancing a biostimulator toward a target site.
FIG. 11 is a diagrammatic view of an operation of implanting a biostimulator at a target site.

### DETAILED DESCRIPTION

Embodiments describe a biostimulator transport system and a biostimulator system for septal pacing. The biostimulator transport system and the biostimulator system may, however, be used in other applications, such as deep brain stimulation. Thus, reference to use of the biostimulator transport system or the biostimulator system for cardiac treatment is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator transport system. Similarly, "proximal" may indicate a second direction opposite to the first direction. Such terms are provided to establish relative frames of reference, however, and are not intended to limit the use or orientation of a biostimulator transport system to a specific configuration described in the various embodiments below.

In an aspect, a biostimulator transport system is adapted to deliver and implant a leadless biostimulator. The biostimulator transport system can include a drive belt to retain the biostimulator during transport and to turn a portion of the biostimulator to drive a pacing electrode of the biostimulator into a target tissue. More particularly, the drive belt can be actuated to rotate the pacing electrode into the target tissue. The belt can be cut to release the biostimulator. Accordingly, the biostimulator can be placed with the pacing electrode in an upper region of a septal wall, and a housing of the biostimulator located near an apex of the right ventricle.

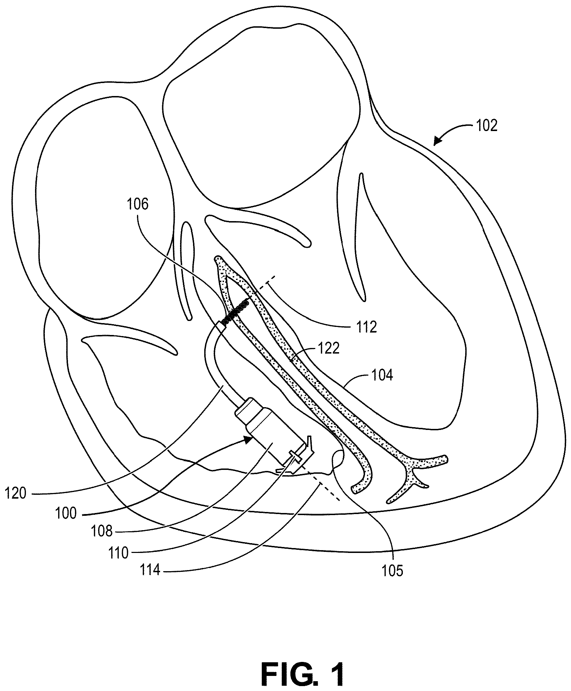

Referring to FIG. 1, a diagrammatic cross-section of a patient heart illustrating an example implantation of a biostimulator in a target anatomy is shown in accordance with an embodiment. A biostimulator system, e.g., a leadless cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to one or more of an interventricular septal wall 104 or a ventricular apex 105 of the heart 102. More particularly, the biostimulator 100 can be delivered to the septum, and one or more elements, such as a pacing electrode 106, can pierce the interventricular septal wall 104 of the septum to engage and anchor the biostimulator 100 to the tissue. Similarly, a housing 108 and/or an anchor 110 can be delivered into the ventricular apex 105.

The pacing electrode 106 can include a helical fixation element having an electrode axis 112. The axis can be directed toward, e.g., normal to, the septal wall when the pacing electrode 106 is affixed to the septal wall. Similarly, the housing 108 can have a housing axis 114, which is directed toward, e.g., oblique to, an apex wall of the ventricular apex 105 when the housing 108 is located therein. When the pacing electrode 106 is affixed to the interventricular septal wall 104, and the housing 108 is located at the ventricular apex 105, the electrode axis 112 can extend in a different direction than the housing axis 114. For example, the electrode axis 112 can extend in a direction that is transverse or oblique to a direction of the housing axis 114. The pacing electrode 106 can be electrically connected to pacing circuitry contained within the housing 108 of the biostimulator 100. Accordingly, the pacing electrode 106 can be located to effectively probe and pace the left bundle branch, while the housing 108 can be placed in a safe and non-obstructive location within the heart chamber.

The non-coaxial relationship of the electrode axis 112 and the housing axis 114 can allow for safe and non-obstructive placement of the pacing electrode 106 and the housing 108. The non-coaxial relationship may be provided by an articulation 120 of the biostimulator 100. The articulation 120 can be located between the pacing electrode 106 and the housing 108. For example, the articulation 120 may be a flexible portion of a lead extension, a hinge, or any other mechanism that acts as a joint or juncture between a distal portion and a proximal portion of the biostimulator. More particularly, the articulation 120 may provide a movable joint between the portions to allow the biostimulator to articulate and conform to the target anatomy.

Leadless pacemakers or other leadless biostimulators can be delivered to or retrieved from a patient using a transport system, e.g., a delivery or retrieval system. The transport system can include catheter-based transport systems used to deliver or retrieve a leadless biostimulator intravenously to or from a patient anatomy. Examples of biostimulator transport systems are described below. In some implementations of biostimulator systems, a biostimulator such as a leadless pacemaker is attached, connected to, or otherwise mounted on a distal end of a catheter of the biostimulator transport system. The leadless pacemaker is thereby advanced intravenously into or out of the heart 102. The biostimulator transport system can include features to engage the leadless pacemaker to allow fixation of the leadless pacemaker to tissue. For example, in implementations where the leadless pacemaker includes an active engaging mechanism, such as a helical fixation element, the biostimulator transport system can include a drive belt configured to engage a portion of the leadless pacemaker and apply torque to screw the active engaging mechanism into or out of the tissue.

When the biostimulator 100 is delivered to and screwed into the septum of the heart 102, the pacing electrode 106 may be positioned for deep septal pacing of a target tissue 122, e.g., a target bundle branch in the septum. For example, an active electrode of the pacing element can be positioned at the left bundle branch in the septum. The biostimulator 100 may deliver pacing impulses through the pacing electrode 106 to the bundle branch(es).

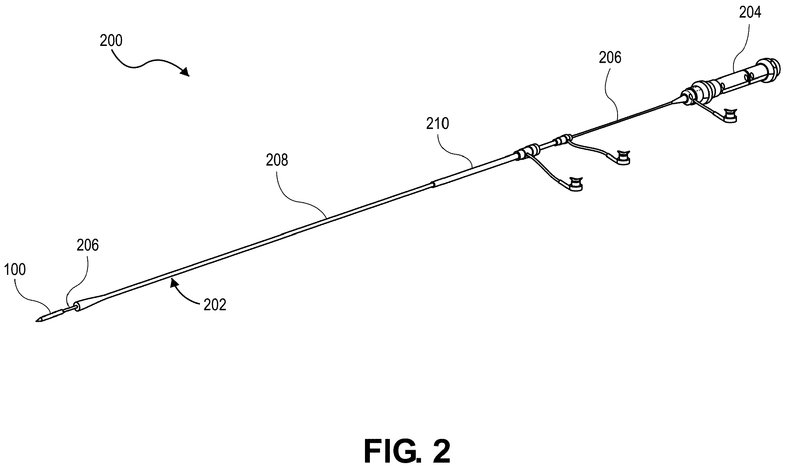

Referring to FIG. 2, a perspective view of a biostimulator system is shown in accordance with an embodiment. The biostimulator system 200 can include a biostimulator transport system 202. The biostimulator transport system 202 can include a handle 204 to control movement and operations of the transport system from outside of a patient anatomy. One or more elongated members extend distally from the handle 204. For example, a support member 206 can extend distally from the handle 204. The support member 206 can extend to a distal end of the transport system 202. In an embodiment, the biostimulator 100 is mounted on the biostimulator transport system 202, e.g., at a distal end of one of the support member 206.

The biostimulator transport system 202 can include a protective sleeve 208 to cover the biostimulator 100 during delivery and implantation. The protective sleeve 208 can extend over, and be longitudinally movable relative to, the support member 206. The biostimulator transport system 202 may also include an introducer sheath 210 that can extend over, and be longitudinally movable relative to, the protective sleeve 208. The introducer sheath 210 can cover a distal end of the protective sleeve 208, the support member 206, and the biostimulator 100 as those components are passed through an access device into the patient anatomy.

Several components of the biostimulator transport system 202 are described above by way of example. It will be appreciated, however, that the biostimulator transport system 202 may be configured to include additional or alternate components. More particularly, the biostimulator transport system 202 may be configured to deliver and/or retrieve the biostimulator 100 to or from the target anatomy. Delivery and/or retrieval of the biostimulator 100 can include retaining the biostimulator 100 during transport to the target anatomy and rotation of the biostimulator 100 during implantation of the biostimulator at the target anatomy. Accordingly, as described below, the biostimulator transport system 202 can incorporate features to retain and rotate the biostimulator 100.

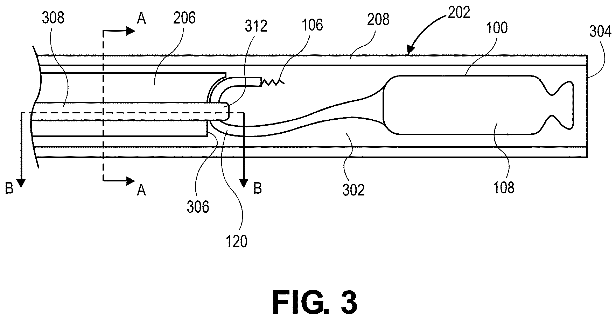

Referring to FIG. 3, a cross-sectional view of a biostimulator retained within an extended sleeve of a biostimulator transport system is shown in accordance with an embodiment. The biostimulator transport system 202 can store and retain the biostimulator 100 within the sleeve 208 during transport to a target anatomy. More particularly, the sleeve 208 can include a sleeve lumen 302, and the biostimulator 100 may be disposed within the sleeve lumen 302 during delivery and/or retrieval to/from the target anatomy. The sleeve 208 can include an elongated tubular member extending from a proximal sleeve end (not shown) to a distal sleeve end 304. The sleeve lumen 302 can extend centrally through a length of the sleeve 208. The distal member end 306 can be a distal tip or face of the elongated shaft. A material, size, and shape of the sleeve 208 may be selected to provide sufficient column strength and/or flexibility to allow the sleeve 208 to be tracked through a vasculature to the target anatomy.

The biostimulator transport system 202 can include the support member 206 extending through the sleeve lumen 302. The support member 206 may be an elongated shaft extending from a proximal member end (not shown) to a distal member end 306. A material, size, and shape of the support member 206 may be selected to provide sufficient column strength and/or flexibility to allow the support member 206 to be tracked through the vasculature to the target anatomy.

In an embodiment, a belt 308 extends longitudinally through the sleeve lumen 302. The belt 308 can be formed from a flexible material, such as an elastomeric material, and can be located between the sleeve 208 and the support member 206. For example, a belt leg may extend longitudinally through the sleeve lumen 302 and be positioned laterally between the support member 206 and a wall of the sleeve 208. The belt 308 can have a belt leg on either side of the support member 206, e.g., a pair of belt legs diametrically opposed within the sleeve lumen 302. The belt legs can extend between the sleeve 208 and the support member 206 to a loop 312 located distal to the distal member end 306. A first belt leg can extend distally beyond the distal member end 306. The belt leg can loop laterally through the sleeve lumen 302 to a second belt leg, which extends back proximally through the sleeve lumen 302. The loop 312 can form a gap between the distal member end 306 and a rear-facing surface of the loop 312 of the belt 308, distal to the distal member end 306.

The biostimulator 100 can be retained within the sleeve lumen 302 by the belt 308. More particularly, a portion of the biostimulator 100, e.g., a portion of the biostimulator between the housing 108 and the pacing electrode 106, can extend through the loop 312. The portion may be the articulation 120 of the biostimulator 100. The articulation 120 can be a flexible portion of a lead extension, for example. The belt 308 can be cinched to hold the portion of the biostimulator 100 against the distal member end 306 and retain the biostimulator 100 relative to the support member 206.

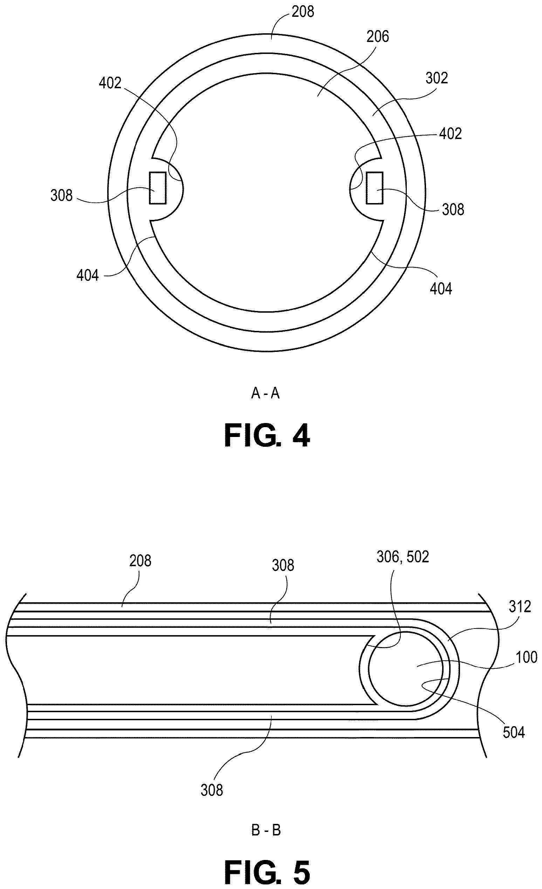

Referring to FIG. 4, a cross-sectional view, taken about line A-A of FIG. 3, of a biostimulator transport system is shown in accordance with an embodiment. The belt 308 of the biostimulator transport system 202 can be guided within a track 402 of the support member 206. The support member 206 may include a lateral surface 404 having the track 402. For example, the lateral surface 404 can have a notch, groove, or another longitudinal depression within which the belt 308 can extend. More particularly, each belt leg of the belt 308 can extend longitudinally through respective tracks 402. The tracks 402 may be diametrically opposed, on opposite lateral surfaces 404 of the support member 206. Thus, the belt legs may be diametrically opposed within the sleeve lumen 302. The belt 308 may have a rectangular cross-sectional area, as shown, or may have a differently shaped cross-sectional area such as a circular cross-sectional area.

The track 402 can maintain a position of the belt legs such that the loop 312 remains centered within the sleeve lumen 302 relative to the distal member end 306. Accordingly, the loop 312 can pull the portion of the biostimulator 100, e.g., the articulation 120, directly against the distal member end 306.

Referring to FIG. 5, a cross-sectional view, taken about line B-B of FIG. 3, of a biostimulator transport system is shown in accordance with an embodiment. In an embodiment, the loop 312 includes a gripping surface 504. The gripping surface 504 can be the rear-facing surface partly defining the gap. The gripping surface 504 can include a rough surface, such as a knurled surface or a tread. Alternatively or additionally, the gripping surface 504 can include a tacky surface, such as a surface coated with a sticky coating. The gripping surface 504 can be on an interior of the belt 308 facing the distal member end 306. Accordingly, the gripping surface 504 can be in contact with the biostimulator 100 when the belt 308 is cinched against the biostimulator 100 to retain the portion of the biostimulator 100 against the distal member end 306.

The gripping surface 504 of the belt 308 can have a higher coefficient of friction than a surface of a socket 502. The biostimulator 100 retained between the distal member end 306 and the belt 308 may therefore more easily slide against the socket 502 than against the belt 308. More particularly, when the belt 308 is pulled over the biostimulator 100, it can grip the biostimulator 100 to cause the biostimulator 100 to rotate between the belt 308 and the socket 502. The belt 308 may therefore be used to drive rotation of the biostimulator 100.

The socket 502 can receive the portion of the biostimulator 100. The socket 502 can include a depression or a hollow that forms a holder for the biostimulator 100. For example, the retained portion of the biostimulator 100 may be a portion of a flexible extension having a circular cross-section, and the socket 502 may have an arched or saddle-shaped depression that conforms to an outer surface of the flexible extension. Similarly, the belt 308 can conform to the outer surface of the flexible extension. Thus, the belt 308 may be cinched to circumferentially support and hold the biostimulator 100 against the distal member end 306.

The support member 206 may have a lubricious surface to facilitate sliding of the belt 308 within the track 402, and sliding of the biostimulator within the socket 502. For example, the support member 206 may be fabricated from a material having low surface friction, e.g., polytetrafluoroethylene, may be coated with a lubricious coating, or may have a surface impregnated with a lubricious material. The smooth surface of the support member 206 can allow the belt 308 to move and be cinched with minimal resistance. The smooth surface can also allow the portion of the biostimulator 100, e.g., the articulation 120, to slide within the socket 520 during implantation of the biostimulator 100.

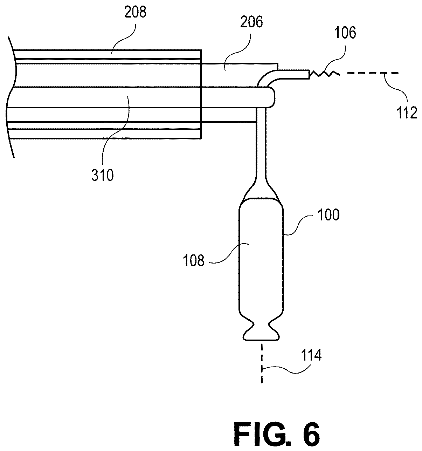

Referring to FIG. 6, a cross-sectional view of a biostimulator exposed from a retracted sleeve of a biostimulator transport system is shown in accordance with an embodiment. The sleeve 208 can be longitudinally movable relative to the support member 206. Accordingly, the sleeve 208 may be retracted relative to the support member 206 to expose the biostimulator 100. When the sleeve 208 is retracted and the biostimulator 100 is exposed, the pacing electrode 106 of the biostimulator 100 may be directed forward. More particularly, the electrode axis 112 may extend longitudinally parallel to a longitudinal axis of the sleeve 208 and the support member 206. The housing 108 of the biostimulator 100, on the other hand, may swing and/or drop downward. For example, the housing axis 114 can extend in a transverse direction, e.g., oblique or orthogonal to the electrode axis 112. Note the difference in relative orientation between the electrode axis 112 and the housing axis 114 with respect to FIG. 3. When the sleeve 208 is in an extended state and the biostimulator 100 is within the sleeve 208, as shown in FIG. 3, the electrode axis 112 can extend in a same direction as the housing axis 114 of the housing 108. By contrast, when the sleeve 208 is in the retracted state and the biostimulator 100 is exposed from the sleeve 208, as shown in FIG. 6, the electrode axis 112 can extend in a different direction than the housing axis 114 of the housing 108.

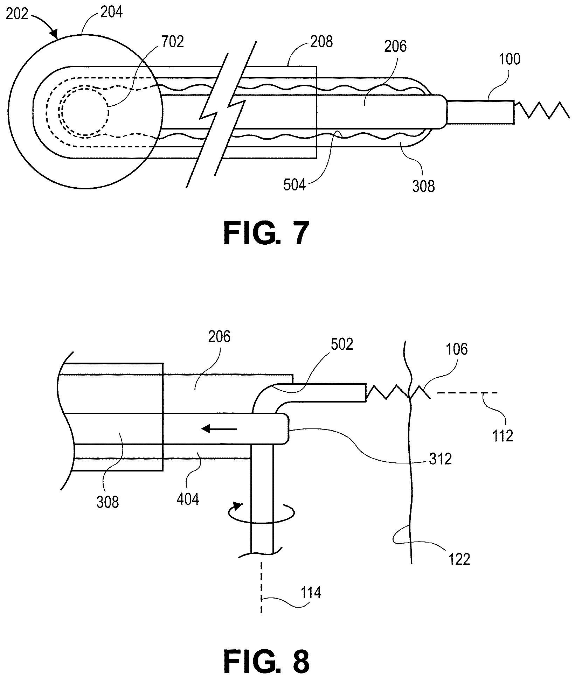

Referring to FIG. 7, a top view of a biostimulator exposed from a retracted sleeve of a biostimulator transport system is shown in accordance with an embodiment. The biostimulator transport system 202 can include a drive mechanism 702 to drive rotation of the biostimulator 100 when the sleeve 208 is retracted and the biostimulator 100 is in the exposed state. In an embodiment, a handle 204 is coupled to the support member 206 and the belt 308. The handle 204 can include the drive mechanism 702, and the drive mechanism may be controlled to drive the belt 308 through the track 402. For example, the drive mechanism 702 can include a knob on the handle 204, which may be rotated by a user. The knob can engage the belt 308, e.g., through one or more transmission components, such as a gear, a friction wheel, or another component. The belt 308 can extend through a length of the sleeve 208 and wrap around the drive mechanism 702 such that the drive mechanism actuates movement of the belt 308. For example, rotation of the knob can cause the belt 308 to slide in the track 402. An interior surface of the belt 308 can include the gripping surface 504, e.g., a tread, such that sliding the belt 308 through the track 402 imparts friction to the biostimulator 100 and causes the biostimulator to spin against the socket 502 of the support member 206.

Referring to FIG. 8, a side view of a biostimulator exposed from a retracted sleeve of a biostimulator transport system is shown in accordance with an embodiment. Actuation of the drive mechanism 702 can cause the belt 308 to slide along the lateral surface 404 of the support member 206. As shown, one of the belt legs can move proximally along the lateral surface 404. The other belt leg, which is hidden behind the support member 206 in FIG. 8, can move distally along an opposing lateral surface 404 of the support member 206. Accordingly, the loop 312 of the belt 308 can move laterally (out of the page). The belt 308 can grip and twist the biostimulator 100, causing the biostimulator to slidably rotate within the socket 502 of the support member 206. In an embodiment, the portion of the biostimulator 100 that is rotated by the belt 308 includes an elongated flexible extension. The extension connects to the pacing electrode 106 and the housing 108 of the biostimulator 100. Accordingly, rotation of the biostimulator 100 can cause a distal portion of the extension, which is connected to the pacing electrode 106, to rotate about the electrode axis 112. Similarly, a proximal portion of the extension, which is connected to the housing 108, can rotate about the housing axis 114. The socket 502 can have a curvature to direct the distal portion of the pacing electrode 106 forward toward the target anatomy. When the pacing electrode 106 is engaged to the target anatomy, rotation of the extension can screw the pacing electrode 106 into the target tissue 122.

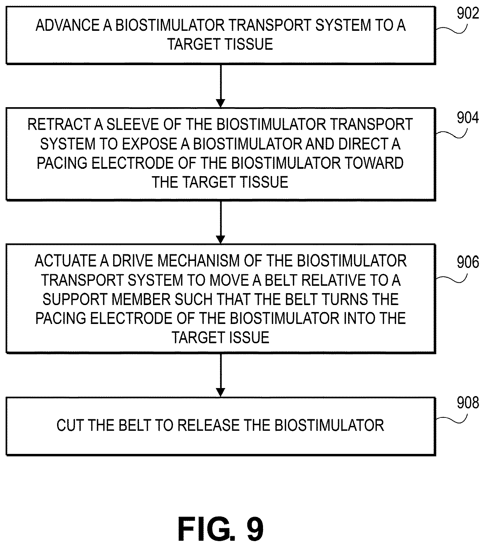

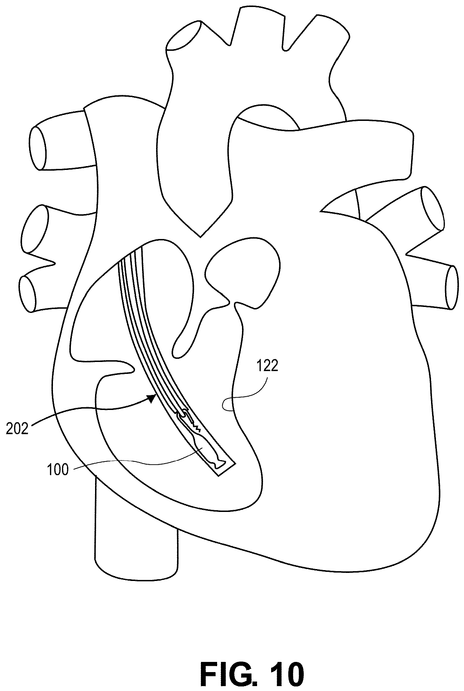

Referring to FIG. 9, a flowchart of a method of implanting a biostimulator 100 for septal pacing is shown. Certain operations of the implantation method are illustrated in FIGS. 10-11. Accordingly, FIGS. 9-11 are described in combination below.

Referring to FIG. 10, a diagrammatic view of an operation of advancing a biostimulator toward a target site is shown. During the implantation procedure, at operation 902, the biostimulator transport system 202 can be advanced to the target tissue 122. More particularly, the biostimulator transport system 202 can carry the biostimulator 100 into the target heart chamber. When implantation is to be within the right ventricle, the biostimulator transport system 202 can be tracked through the inferior vena cava into the right atrium and across the tricuspid valve into the right ventricle. The distal end of the biostimulator transport system 202 can be steered toward a desired location of the septal wall 104. For example, the target area may be in an upper region of the interventricular septal wall 104.

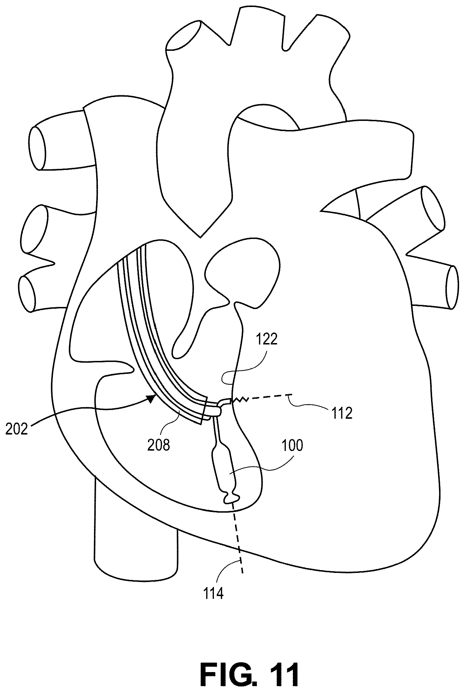

Referring to FIG. 11, a diagrammatic view of an operation of implanting a biostimulator at a target site is shown. At operation 904, the sleeve 208 of the biostimulator transport system 202 is retracted to expose the biostimulator 100. In the exposed state, the electrode axis 112 and the housing axis 114 can move from an aligned orientation (as shown in FIGS. 3 and 10) to a misaligned orientation (as shown in FIG. 6). In the misaligned orientation, the electrode axis 112 may be directed toward the target tissue 122 and the housing axis 114 can be directed downward toward an apex of the heart chamber.

The pacing electrode 106 of the biostimulator 100 can be directed toward the target tissue 122. For example, biostimulatorr transport system 202 can include a steering mechanism in the handle 204 to allow a user to deflect and/or steer the distal member end 306 toward the target tissue 122. Steering may be achieved, for example, using a steering cable or by incorporating a fixed curve into the catheter. Other steering mechanisms may be used. When the catheter is steered, the pacing electrode 106 can be directed toward the target tissue 122.

The pacing electrode 106 may be affixed to the target tissue 122, e.g., the interventricular septum. In operation 906, the drive mechanism 702 is actuated to drive the pacing electrode 106 into the target tissue 122. Actuation of the drive mechanism 702 can include moving the belt 308, e.g., by spinning a knob that drives the belt 308, relative to the support member 206. The belt 308 may grip and rotate the biostimulator 100 against the distal member end 306, causing the belt 308 to turn the pacing electrode 106 of the biostimulator 100 into the target tissue 122. More particularly, the knob on the handle 204 can be rotated to impart movement to the belt 308, and thus, when a distal end of the biostimulator 100 is in contact with the septal wall 104, torque can be transferred from the biostimulator transport system 202 to the biostimulator 100 to drive the pacing electrode 106 (which may include a fixation helix) into the target tissue 122. The pacing electrode 106 and/or the fixation helix can be screwed into the tissue to a desired depth by rotating the helically-shaped electrode into the target tissue 122. In an embodiment, the pacing electrode 106 may engage the tissue at a depth that allows effective pacing of the target bundle branch.

When the biostimulator 100 is implanted in the desired location, the biostimulator transport system 202 may release the biostimulator 100. For example, at operation 908, the belt 308 may be cut to release the biostimulator 100. The biostimulator transport system 202 can incorporate a blade to perform the cutting, or a user may use a separate cutting tool, such as scissors, to cut the belt 308. When the belt 308 is cut, the biostimulator transport system 202 can be retracted. The belt 308 will slide over and release from the biostimulator 100 during system retraction, and the biostimulator 100 can remain in position at the target tissue 122. The biostimulator 100 may therefore remain in place to perform the pacing function.

The present invention is set out in the claims that follow:

## Claims

1. A biostimulator transport system (202), comprising:
a sleeve (208) having a sleeve lumen (302);
a support member (206) extending through the sleeve lumen (302) to a distal member end (306); and
a belt (308) having a first leg laterally between a first lateral surface of the support member (206) and the sleeve (208), and a second leg laterally between a second lateral surface of the support member (206) and the sleeve (208), wherein the first leg and the second leg extend longitudinally through the sleeve lumen (302) to a loop (312) distal to the distal member end (306), to retain a biostimulator between the belt (308) and the support member (206) within the sleeve lumen (302).

2. The biostimulator transport system of claim 1, wherein the sleeve (208) is longitudinally moveable relative to the support member (206).

3. The biostimulator transport system of claim 1 or 2, wherein the distal member end (306) has a socket (502) configured to receive a portion of a biostimulator (100).

4. The biostimulator transport system of any one of claims 1 to 3, wherein the support member (206) has a lubricious surface.

5. The biostimulator transport system of any one of claims 1 to 4, wherein the first lateral surface of the support member (206) has a track (402), and wherein the belt (308) extends longitudinally through the track (402).

6. The biostimulator transport system of claim 5, further comprising a handle (204) coupled to the support member (206) and the belt (308), wherein the handle (204) includes a drive mechanism (702) configured to drive the belt (308) through the track (402).

7. The biostimulator transport system of claim 6, wherein the drive mechanism (702) includes a knob, and wherein rotation of the knob causes the belt (308) to slide in the track (402).

8. The biostimulator transport system of any one of claims 1 to 7, wherein the loop (312) has a gripping surface (504) facing the distal member end (306).

9. The biostimulator transport system of claim 8, wherein the gripping surface (504) includes a rough surface.

10. The biostimulator transport system of claim 8, wherein the gripping surface (504) includes a tacky surface.

11. A biostimulator system (200), comprising:
a biostimulator (100) including a pacing electrode (106) electrically connected to pacing circuitry contained within a housing (108) of the biostimulator (100); and
a biostimulator transport system (202) of any one of claims 1 to 10.

12. The biostimulator system of claim 11, wherein a portion of the biostimulator (100) between the pacing electrode (106) and the housing (108) extends through the loop (312).

## Patentansprüche

1. Biostimulatortransportsystem (202), Folgendes umfassend:
eine Hülse (208), die ein Hülsenlumen (302) aufweist;
ein Stützelement (206), das sich durch das Hülsenlumen (302) zu einem distalen Elementende (306) erstreckt; und
einen Riemen (308), der einen ersten Schenkel, der sich seitlich zwischen einer ersten Seitenfläche des Stützelements (206) und der Hülse (208) befindet, und einen zweiten Schenkel, der sich seitlich zwischen einer zweiten Seitenfläche des Stützelements (206) und der Hülse (208) befindet, aufweist, wobei sich der erste Schenkel und der zweite Schenkel in Längsrichtung durch das Hülsenlumen (302) zu einer Schleife (312) distal zu dem distalen Elementende (306) erstrecken, um einen Biostimulator zwischen dem Riemen (308) und dem Stützelement (206) innerhalb der Hülsenlumens (302) zu halten.

2. Biostimulatortransportsystem nach Anspruch 1, wobei die Hülse (208) relativ zu dem Stützelement (206) in Längsrichtung bewegbar ist.

3. Biostimulatortransportsystem nach Anspruch 1 oder 2, wobei das distale Elementende (306) eine Buchse (502) aufweist, die konfiguriert ist, um einen Abschnitt eines Biostimulators (100) aufzunehmen.

4. Biostimulatortransportsystem nach einem der Ansprüche 1 bis 3, wobei das Stützelement (206) eine gleitfähige Fläche aufweist.

5. Biostimulatortransportsystem nach einem der Ansprüche 1 bis 4, wobei die erste Seitenfläche des Stützelements (206) eine Bahn (402) aufweist und wobei sich der Riemen (308) in Längsrichtung durch die Bahn (402) erstreckt.

6. Biostimulatortransportsystem nach Anspruch 5, ferner umfassend einen Griff (204), der mit dem Stützelement (206) und dem Riemen (308) gekoppelt ist, wobei der Griff (204) einen Antriebsmechanismus (702) beinhaltet, der konfiguriert ist, um den Riemen (308) durch die Bahn (402) anzutreiben.

7. Biostimulatortransportsystem nach Anspruch 6, wobei der Antriebsmechanismus (702) einen Knopf beinhaltet und wobei eine Drehung des Knopfes bewirkt, dass der Riemen (308) in der Bahn (402) gleitet.

8. Biostimulatortransportsystem nach einem der Ansprüche 1 bis 7, wobei die Schleife (312) eine Greiffläche (504) aufweist, die dem distalen Elementende (306) zugewandt ist.

9. Biostimulatortransportsystem nach Anspruch 8, wobei die Greiffläche (504) eine raue Oberfläche beinhaltet.

10. Biostimulatortransportsystem nach Anspruch 8, wobei die Greiffläche (504) eine haftende Oberfläche beinhaltet.

11. Biostimulatorsystem (200), Folgendes umfassend:
einen Biostimulator (100), der ceine Schrittmacherelektrode (106) beinhaltet, die elektrisch mit einem Schrittmacherschaltkreis verbunden ist, der in einem Gehäuse (108) des Biostimulators (100) enthalten ist; und
ein Biostimulatortransportsystem (202) nach einem der Ansprüche 1 bis 10.

12. Biostimulatorsystem nach Anspruch 11, wobei sich ein Abschnitt des Biostimulators (100) zwischen der Schrittmacherelektrode (106) und dem Gehäuse (108) durch die Schleife (312) erstreckt.

## Revendications

1. Système de transport de biostimulateur (202), comprenant :
un manchon (208) ayant un lumen de manchon (302) ;
un élément de support (206) s'étendant à travers le lumen de manchon (302) vers une extrémité d'élément distale (306) ; et
une courroie (308) ayant une première jambe latéralement entre une première surface latérale de l'élément de support (206) et le manchon (208), et une seconde jambe latéralement entre une seconde surface latérale de l'élément de support (206) et le manchon (208), dans lequel la première jambe et la seconde jambe s'étendent longitudinalement à travers le lumen de manchon (302) vers une boucle (312) distale par rapport à l'extrémité d'élément distale (306), afin de retenir un biostimulateur entre la courroie (308) et l'élément de support (206) à l'intérieur du lumen de manchon (302).

2. Système de transport de biostimulateur selon la revendication 1, dans lequel le manchon (208) peut se déplacer longitudinalement par rapport à l'élément de support (206).

3. Système de transport de biostimulateur selon la revendication 1 ou 2, dans lequel l'extrémité d'élément distale (306) a une prise (502) configurée pour recevoir une partie d'un biostimulateur (100).

4. Système de transport de biostimulateur selon l'une quelconque des revendications 1 à 3, dans lequel l'élément de support (206) a une surface lubrifiante.

5. Système de transport de biostimulateur selon l'une quelconque des revendications 1 à 4, dans lequel la première surface latérale de l'élément de support (206) a une piste (402), et dans lequel la courroie (308) s'étend longitudinalement à travers la piste (402).

6. Système de transport de biostimulateur selon la revendication 5, comprenant en outre une poignée (204) accouplée à l'élément de support (206) et à la courroie (308), dans lequel la poignée (204) comporte un mécanisme d'entraînement (702) configuré pour entraîner la courroie (308) à travers la piste (402).

7. Système de transport de biostimulateur selon la revendication 6, dans lequel le mécanisme d'entraînement (702) comporte un bouton, et dans lequel la rotation du bouton amène la courroie (308) à glisser dans la piste (402).

8. Système de transport de biostimulateur selon l'une quelconque des revendications 1 à 7, dans lequel la boucle (312) a une surface de préhension (504) faisant face à l'extrémité d'élément distale (306).

9. Système de transport de biostimulateur selon la revendication 8, dans lequel la surface de préhension (504) comporte une surface rugueuse.

10. Système de transport de biostimulateur selon la revendication 8, dans lequel la surface de préhension (504) comporte une surface collante.

11. Système de biostimulateur (200), comprenant:
un biostimulateur (100) comportant une électrode de stimulation (106) connectée électriquement à une circuiterie de stimulation contenue à l'intérieur d'un boîtier (108) du biostimulateur (100) ; et
un système de transport de biostimulateur (202) selon l'une quelconque des revendications 1 à 10.

12. Système de biostimulateur selon la revendication 11, dans lequel une partie du biostimulateur (100) entre l'électrode de stimulation (106) et le boîtier (108) s'étend à travers la boucle (312).
